# EUROPEAN PATENT APPLICATION

(11) **EP 1 557 173 A1**
(43) Date of publication of application: **27.07.2005**
(21) Application number: 04014530.2
(22) Date of filing: 29.01.1998
(51) Int. Cl.: A61K 38/16, C12P 21/02, C12N 15/00, C12N 15/01, C12N 15/09, C12N 15/12, C12N 15/63, C12N 15/70, C12N 15/79, C07K 14/705

(54) **Soluble CTLA4 mutant molecules and uses thereof**

(30) Priority: 31.01.1997 US 36594 P
(62) Divisional of application: 98903873.2
(71) Applicant: BRISTOL-MYERS SQUIBB COMPANY, Princeton, NJ 08543-4000 (US)
(72) Inventor: Peach, Robert James, San Diego, CA 92129 (US); Naemura, Joseph R., Bellevue, WA 98005 (US); Linsley, Peter S., Washington, 98119 Seattle (US); Bajorath, Jürgen, Lynwood, CA 98037 (US)
(74) Representative: Reitstötter - Kinzebach & Partner (GbR)

(57) **Abstract**

The present invention relates to soluble CTLA4 mutant molecules having a higher avidity for CD86 than wildtype CTLA4, pharmaceutical compositions comprising said soluble CTLA4 mutant molecules, immunosuppressive combinations comprising said soluble CTLA4 mutant molecules and at least one further immunosuppressant, and the use of said soluble CTLA4 mutant molecules for immune suppression, for treating immune system diseases and, in combination with a ligand reactive with IL-4, for inhibiting graft versus host disease (GVHD). Also, the invention relates to in vitro methods for regulating functional CTLA4 and CD28 positve T cell interactions with CD80 and/or CD86 positive cells.

The present invention also relates to nucleic acid molecules encoding the amino acid sequence corresponding to said CTLA4 mutant molecules, plasmids comprising said nucleic acid molecules, and host vector systems comprising said plasmids.

Further, the present invention relates to methods for producing a protein comprising growing said host vector system, and proteins obtainable by said methods.

## Description

Throughout this application various publications are referenced. The disclosures of these publications in their entireties are hereby incorporated by reference into this application in order to more fully describe the state of the art to which this invention pertains.

### BACKGROUND OF THE INVENTION

Antigen-nonspecific intercellular interactions between T-lymphocytes and antigen-presenting cells (APCs) generate T cell costimulatory signals that generate T cell responses to antigen (Jenkins and Johnson (1993) Curr. Opin. Immunol. 5:361-367). Costimulatory signals determine the magnitude of a T cell response to antigen, and whether this response activates or inactivates subsequent responses to antigen (Mueller et al. (1989) Annu. Rev. Immunol. 7: 445-480).

T cell activation in the absence of costimulation results in an aborted or anergic T cell response (Schwartz, R.H. (1992) Cell 71:1065-1068). One key costimulatory signal is provided by interaction of T cell surface receptors CD28 and CTLA4 with B7 (also known as B7-1 and B7-2, or CD80 and CD86, respectively) related molecules on APC (P. Linsley and J. Ledbetter (1993) Annu. Rev. Immunol. 11:191-212).

The molecule now known as CD80 (B7-1) was originally described as a human B cell-associated activation antigen (Yokochi, T. et al. (1981) J. Immunol. 128:823-827; Freeman, G.J. et al. (1989) J. Immunol. 143:2714-2722), and subsequently identified as a counterreceptor for the related T cell molecules CD28 and CTLA4 (Linsley, P., et al. (1990) PNAS USA 87:5031-5035; Linsley, P.S. et al. (1991a) J. Exp. Med. 173:721-730; Linsley, P.S. et al. (1991b) J. Exp. Med. 174:561-570).

More recently, another counterreceptor for CTLA4Ig was identified on antigen presenting cells (APC) (Azuma, N. et al. (1993) Nature 366:76-79; Freeman (1993a) Science 262:909-911; Freeman, G.J. et al. (1993b) J. Exp. Med. 178:2185-2192; Hathcock, K.L.S., et al. (1994) J. Exp. Med. 180: 631-640; Lenschow, D.J. et al., (1993) PNAS USA 90:11054-11058; Ravi-Wolf, Z., et al. (1993) PNAS USA 90:11182-11186; Wu, Y. et al. (1993) J. Exp. Med. 178:1789-1793).

This molecule, now known as CD86 (Caux, C., et al. (1994) J. Exp. Med. 180:1841-1848), but also called B7-0 (Azuma et al., 1993, supra) or B7-2 (Freeman et al., 1993a, supra), shares about 25% sequence identity with CD80 in its extracellular region (Azuma et al., 1993, supra, Freeman et al., 1993a, supra, 1993b, supra). CD86-transfected cells trigger CD28-mediated T cell responses (Azuma et al., 1993, supra; Freeman et al., 1993a, 1993b, supra).

Comparisons of expression of CD80 and CD86 have been the subject of several studies (Azuma et al. 1993, supra; Hathcock et al., 1994 supra; Larsen, C.P., et al.(1994) J. Immunol. 152:5208-5219; Stack, R.M., et al., (1994) J. Immunol. 152:5723-5733). Current data indicate that expression of CD80 and CD86 are regulated differently, and suggest that CD86 expression tends to precede CD80 expression during an immune response.

Soluble forms of CD28 and CTLA4 have been constructed by fusing variable (v)-like extracellular domains of CD28 and CTLA4 to immunoglobulin (Ig) constant domains resulting in CD28Ig and CTLA4Ig. CTLA4Ig binds both CD80+ and CD86+ cells more strongly than CD28Ig (Linsley, P. et al. (1994) Immunity 1:793-80). Many T cell-dependent immune responses are blocked by CTLA4Ig both *in vitro and in vivo.* (Linsley, et al., (1991b), supra; Linsley, P.S. et al., (1992a) Science 257:792-795; Linsley, P. S. et al., (1992b) J. Exp. Med. 176:1595-1604; Lenschow, D.J. et al. (1992), Science 257:789-792; Tan, P. et al., (1992) J. Exp. Med. 177:165-173; Turka, L.A., (1992) PNAS USA 89:11102-11105).

Peach et al., (J. Exp. Med. (1994) 180:2049-2058) identified regions in the CTLA4 extracellular domain which are important for strong binding to CD80. Specifically, a hexapeptide motif (MYPPPY) in the complementarity determining region 3 (CDR3)-like region was identified as fully conserved in all CD28 and CTLA4 family members. Alanine scanning mutagenesis through the motif in CTLA4 and at selected residues in CD28Ig reduced or abolished binding to CD80.

Chimeric molecules interchanging homologous regions of CTLA4 and CD28 were also constructed. Molecules HS4, HS4-A and HS4-B were constructed by grafting CDR3-like regions of CTLA4 which also included a portion carboxy terminally extended to include certain nonconserved amino acid residues onto CD28Ig. These homologue mutants showed higher binding avidity to CD80 than did CD28.

In another group of chimeric homologue mutants, the CDR1-like region of CTLA4, which is not conserved in CD28 and is predicted to be spatially adjacent to the CDR3-like region was grafted, into HS4 and HS4-A. These chimeric homologue mutant molecules (designated HS7 and HS8) demonstrated even greater binding avidity for CD80.

Chimeric homologue mutant molecules were also made by grafting into HS7 and HS8 the CDR2-like region of CTLA4, but this combination did not further improve the binding avidity for CD80. Thus, the MYPPPY motif of CTLA4 and CD28 were determined to be critical for binding to CD80, but certain non-conserved amino acid residues in the CDR1-and CDR3-like regions of CTLA4 were also responsible for increased binding avidity of CTLA4 with CD80.

CTLA4Ig was shown to effectively block CD80-associated T cell co-stimulation but was not as effective at blocking CD86-associated responses. Soluble CTLA4 mutant molecules having a higher avidity for CD86 than wild type CTLA4 were constructed as possibly better able to block the priming of antigen specific activated cells than CTLA4Ig.

Site-directed mutagenesis and a novel screening procedure were used to identify several mutations in the extracellular domain of CTLA4 that preferentially improve binding avidity for CD86. These molecules will provide better pharmaceutical compositions for immune suppression and cancer treatment than previously known soluble forms of CTLA4.

### SUMMARY OF THE INVENTION

The invention provides soluble CTLA4 mutant molecules which bind with greater avidity to the CD86 antigen than wildtype CTLA4.

In one embodiment, the CTLA4 mutant molecule is designated LEA29Y. LEA29Y binds -2-fold more avidly than wildtype CTLA4Ig (hereinafter referred to as CTLA4Ig) to CD86. This stronger binding results in LEA29Y being up to 10-fold more effective than CTLA4Ig at blocking immune responses.

In another embodiment, the CTLA4 mutant molecule is designated L106E. L1063 also binds more avidly than CTLA4Ig to CD86.

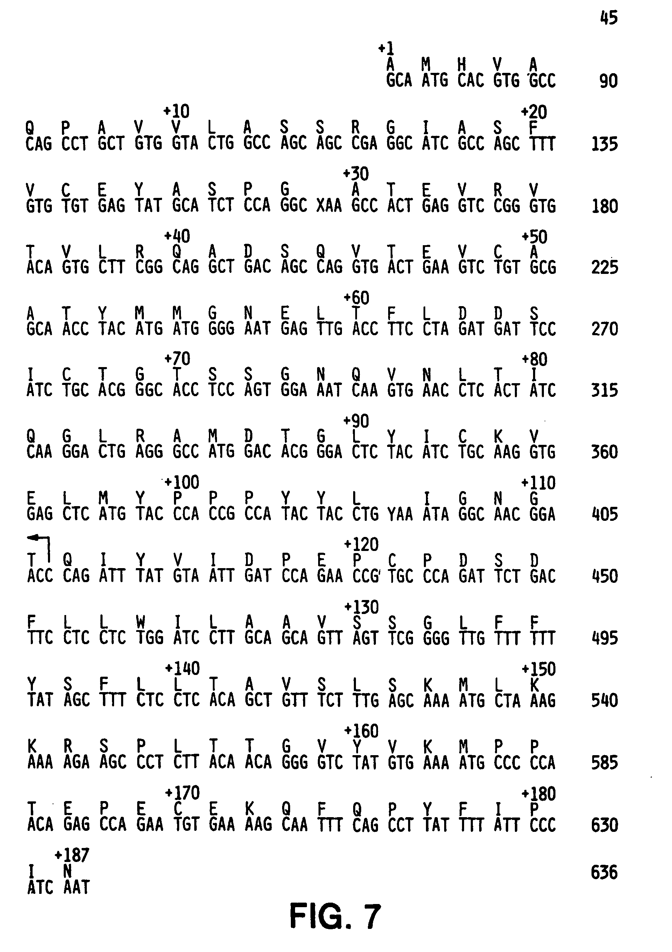

The present invention also provides a soluble CTLA4 mutant molecule which binds CD86, the CTLA4 mutant molecule having an amino acid sequence shown in Figure 7, wherein the amino acid at position 29 designated Xaa is selected from the group consisting of alanine and tyrosine, and wherein the amino acid at position 106 designated Yaa is selected from the group consisting of glutamic acid, asparagine, aspartic acid, glutamine, isoleucine, leucine, and threonine. Specifically, Xaa is alanine and Yaa is glutamic acid or Xaa is tyrosine and Yaa is glutamic acid. According to a particular embodiment, the soluble CTLA4 mutant molecule comprises the 187 amino acids shown in SEQ ID NO 1 beginning with alanine at position 1 and ending with asparagine at position 187.

The present invention also provides a soluble CTLA4 mutant molecule having (a) a first amino acid sequence corresponding to the extracellular domain of CTLA4 mutant as shown in Figure 7; and (b) a second amino acid sequence corresponding to a moiety that alters the solubility, affinity and/or valency of the CTLA4 mutant molecule for binding to the CD86 antigen. Specifically, the moiety is an immunoglobulin constant region.

The present invention also provides a soluble mutant CTLA4Ig fusion protein reactive with the CD86 antigen, said protein having a first amino acid sequence consisting of the extracellular domain of CTLA4 mutant as shown in Figure 7 and a second amino acid sequence consisting of the hinge, CH2 and CH3 regions of human immunoglobulin Cy1.

The present invention also provides a soluble CTLA4 mutant receptor protein having the amino acid sequence depicted in Figure 7 which recognizes and binds a CD86 antigen.

The present invention also provides a soluble CTLA4 mutant molecule comprising the 187 amino acids shown in SEQ ID NO 1 beginning with alanine at position 1 and ending with asparagine at position 187.

The present invention provides further a nucleic acid molecule, specifically a cDNA, encoding the amino acid sequence corresponding to the soluble mutant CTLA4; a plasmid which comprises the cDNA; and a host vector system comprising the plasmid in a suitable host cell. Specifically, the suitable host cell is a bacterial cell or a eucaryotic cell.

The present invention also provides a method for producing a protein comprising growing the host vector system so as to product the protein in the host and recovering the protein so produced.

The present invention also provides a method for regulating functional CTLA4 positive T cell interactions with CD80 and CD86 positive cells comprising contacting the CD80 and CD86 positive cells with the soluble CTLA4 mutant molecule so as to form CTLA4/CD80 and/or CTLA4/CD86 complexes, the complexes interfering with reaction of endogenous CTLA4 antigen with CD80 and CD86. Specifically, the soluble CTLA4 mutant molecule is a fusion protein that contains at least a portion of the extracellular domain of mutant CTLA4. More specifically, the soluble CTLA4 mutant molecule is CTLA4Ig fusion protein having a first amino acid sequence containing amino acid residues from about position 1 to about position 125 of the amino acid sequence corresponding to the extracellular domain of CTLA4 and a second amino acid sequence containing amino acid residues corresponding to the hinge, CH2 and CH3 regions of human immunoglobulin Cy1 as shown in SEQ ID NO 1. According to a particular embodiment, the CD86 positive cells are contacted with fragments or derivatives of the soluble CTLA4 mutant molecule. In particular, the CD86 positive cells are B cells. In particular, the interaction of the CTLA4-positive T cells with the CD80 and CD86 positive cells is inhibited.

The present invention also provides a method for treating immune system diseases mediated by T cell interactions with CD80 and CD86 positive cells comprising administering to a subject the soluble CTLA4 mutant molecule to regulate T cell interactions with the CD86 positive cells. Specifically, the soluble CTLA4 mutant molecule is CTLA4Ig fusion protein or a mutant CD28Ig/CTLA4Ig fusion protein hybrid. In particular, said T cell interactions are inhibited.

The present invention also provides a method for inhibiting graft versus host disease in a subject which comprises administering to the subject the soluble CTLA4 mutant molecule and a ligand reactive with IL-4.

### BRIEF DESCRIPTION OF THE FIGURES

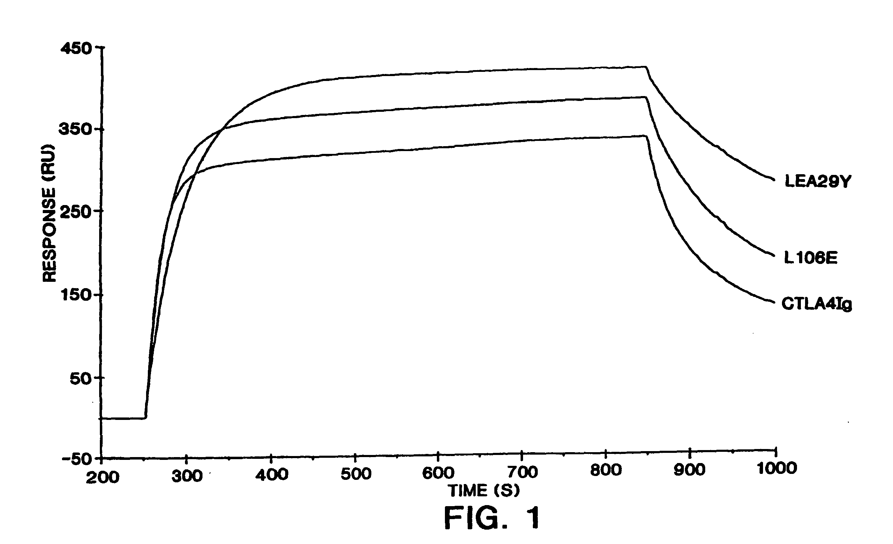

Figure 1: Equilibrium binding analysis of LEA29Y, L106E, and wild-type CTLA4Ig to CD86Ig. LEA29Y binds more strongly to CD86Ig than does L106E or CTLA4Ig. Equilibrium binding constants (Kd) were determined and shown in Table 1. The lower Kd of LEA29Y (2.6) than L106E (3.4) or CTLA4Ig (5.2) indicates higher binding avidity to CD86Ig. All three molecules have similar equilibrium binding constants to CD80Ig.
Figure 2: FACS assay showing LEA29Y and L106E bind more strongly to CHO cells stably transfected with human CD86 than does CTLA4Ig. Binding of each protein to human CD80-transfected CHO cells is equivalent.
Figure 3: *In vitro* functional assays showing that LEA29Y is ∼ 10-fold more effective than CTLA4Ig at inhibiting proliferation of CD86 + PMA treated human T cells. Inhibition of CD80 + PMA stimulated proliferation by CTLA4Ig and LEA29Y is more equivalent.
Figure 4: LEA29Y is -10-fold more effective than CTLA4Ig at inhibiting IL-2, IL-4, and K-interferon cytokine production of allostimulated human T cells.
Figure 5: LEA29Y is 5-7-fold more effective than CTLA4Ig at inhibiting IL-2, IL-4, and K-interferon cytokine production of allostimulated human T cells.
Figure 6: LEA29Y is -10-fold more effective than CTLA4Ig at inhibiting proliferation of PHA-stimulated monkey PBMC's.
Figure 7: depicts the complete amino acid sequence encoding a soluble CTLA4 molecule.

### DETAILED DESCRIPTION OF THE INVENTION

### DEFINITION

As used in this application, the following words or phrases have the meanings specified.

As used herein "CTLA4 mutant molecule" is a molecule having at least an extracellular domain of CTLA4 or any portion thereof which recognizes and binds CD86. The molecule is mutated so that it exhibits a higher avidity for CD86 than wildtype CTLA4. It may include a biologically or chemically active non-CTLA4 molecule therein or attached thereto. The molecule may be soluble (i.e., circulating) or bound to a surface.

As used herein "wildtype CTLA4" is naturally occurring CTLA4 or the CTLA4Ig described in Linsley et al. (1989), supra.

In order that the invention herein described may be more fully understood, the following description is set forth.

### COMPOSITIONS OF THE INVENTION

The invention provides soluble CTLA4 mutant molecules which bind with a higher avidity to CD86 than CTLA4Ig. Soluble CTLA4 mutant molecules having a higher avidity for CD86 than wild type CTLA4 should be better able to block the priming of antigen specific activated cells than CTLA4Ig.

In one embodiment of the invention, the soluble CTLA4 mutant molecule has an amino acid sequence shown in Figure 7. Specifically, the amino acid at position 29 designated Xaa is selected from the group consisting of alanine, leucine, phenylalanine, tryptophan and tyrosine. Further, the amino acid at position 106 designated Yaa is selected from the group consisting of glutamic acid and leucine.

In another embodiment, the soluble CTLA4 mutant molecule comprises the 187 amino acids shown in SEQ ID NO 1 beginning with alanine at position 1 and ending with asparagine at position 187. In that embodiment Xaa is tyrosine and Yaa is glutamic acid (designated herein as LEA29Y). Alternatively, Xaa is alanine and Yaa is glutamic acid (designated herein as L106E).

The invention further provides a soluble CTIA4 mutant molecule having a first amino acid sequence corresponding to the extracellular domain of CTLA4 mutant as shown in Figure 7 and a second amino acid sequence corresponding to a moiety that alters the solubility, affinity and/or valency of the CTLA4 mutant molecule for binding to the CD86 antigen.

In accordance with the practice of the invention, the moiety can be an immunoglobulin constant region or portion thereof. For in vivo use, it is preferred that the immunoglobulin constant region does not elicit a detrimental immune response in the subject. For example, in clinical protocols, it is preferred that mutant molecules include human or monkey immunoglobulin constant regions. One example of a suitable immunoglobulin region is human C(gamma)1. Other isotypes are possible. Further, other weakly or non-immunogenic immunoglobulin constant regions are possible.

The invention further provides soluble mutant CTLA4Ig fusion proteins preferentially reactive with the CD86 antigen compared to wildtype CTLA4, the protein having a first amino acid sequence consisting of the extracellular domain of CTLA4 mutant as shown in Figure 7 and a second amino acid sequence consisting of the hinge, CH2 and CH3 regions of a human immunoglobulin, e.g., Cγ1.

The present invention also provides a soluble CTLA4 mutant receptor protein having the amino acid sequence depicted in Figure 7(SEQ ID NO: 1) which preferentially recognizes and binds CD86 with an avidity of at least five times that of wild type CTLA4.

Additionally, the invention provides a soluble CTLA4 mutant molecule comprising the 187 amino acids shown in SEQ ID NO 1 beginning with alanine at position 1 and ending with asparagine at position 187.

Further, the invention provides a soluble CTLA4 mutant molecule having (a) a first amino acid sequence of a membrane glycoprotein, e.g., CD28, CD86, CD80, CD40, and gp39, which blocks T cell proliferation fused to a second amino acid sequence; (b) the second amino acid sequence being a fragment of the extracellular domain of mutant CTLA4 which blocks T cell proliferation as shown in Figure 7; and (c) a third amino acid sequence which acts as an identification tag or enhances solubility of the molecule. For example, the third amino acid sequence can consist essentially of amino acid residues of the hinge, CH2 and CH3 regions of a non-immunogenic immunoglobulin molecule. Examples of suitable immunoglobulin molecules include but are not limited to human or monkey immunoglobulin, e.g., C(gamma)1. Other isotypes are possible.

Mutant CTLA4 (also used herein as CTLA4 mutant molecule) can be rendered soluble by joining a second molecule. The second molecule can function to enhance solubility of CTLA4 or as identification tags. Examples of suitable second molecules include but are not limited to p97 molecule, env gp120 molecule, E7 molecule, and ova molecule (Dash, B. et al. J. Gen. Virol. 1994 June, 75 (Pt 6):1389-97; Ikeda, T., et al. Gene, 1994 Jan 28, 138(1-2):193-6; Falk, K., et al. Cell. Immunol. 1993 150(2):447-52; Fujisaka, K. et al. Virology 1994 204(2):789-93). Other molecules are possible (Gerard, C. et al. Neuroscience 1994 62(3):721; Byrn, R. et al. 1989 63(10):4370; Smith, D. et al. Science 1987 238:1704; Lasky, L. Science 1996 233:209).

The invention further provides nucleic acid molecules encoding the amino acid sequence corresponding to the soluble mutant CTLA4 molecules of the invention. In one embodiment, the nucleic acid molecule is a DNA (e.g., CDNA) or a hybrid thereof. Alternatively, the molecules is RNA or a hybrid thereof.

Additionally, the invention provides a plasmid which comprises the cDNA of the invention. Also, a host vector system is provided. This system comprises the plasmid of invention in a suitable host cell. Examples of suitable host cells include but are not limited to bacterial cells and eucaryotic cells.

The invention further provides methods for producing a protein comprising growing the host vector system of the invention so as to produce the protein in the host and recovering the protein so produced.

Additionally, the invention provides a method for regulating functional CTLA4 and CD28 positive T cell interactions with CD86 and/or CD80 positive cells. The method comprises contacting the CD80 and/or CD86 positive cells with the soluble CTLA4 mutant molecule of the invention so as to form CTLA4/CD80 and/or CTLA4/CD86 complexes, the complexes interfering with reaction of endogenous CTLA4 antigen with CD80 and/or CD86. In one embodiment of the invention, the soluble CTLA4 mutant molecule is a fusion protein that contains at least a portion of the extracellular domain of mutant CTLA4. In another embodiment, the soluble CTLA4 mutant molecule is CTLA4Ig fusion protein having a first amino acid sequence containing amino acid residues from about position 1 to about position 125 of the amino acid sequence corresponding to the extracellular domain of CTLA4 and a second amino acid sequence containing amino acid residues corresponding to the hinge, CH2 and CH3 regions of human immunoglobulin gamma, e.g., cγ1 as shown in SEQ ID NO 1.

In accordance with the practice of the invention, the CD86 positive cells are contacted with fragments or derivatives of the soluble CTLA4 mutant molecule. Alternatively, the soluble CTLA4 mutant molecule is a CD28Ig/CTLA4Ig fusion protein hybrid having a first amino acid sequence corresponding to a portion of the extracellular domain of CD28 receptor fused to a second amino acid sequence corresponding to a portion of the extracellular domain of CTLA4 mutant receptor (SEQ ID NO 1) and a third amino acid sequence corresponding to the hinge, CH2 and CH3 regions of human immunoglobulin Cγ1.

The present invention further provides a method for treating immune system diseases mediated by CD28 and/or CTLA4 positive cell interactions with dendritic cells with CD86/CD80 positive cells. In one embodiment, T cell interactions are inhibited.

This method comprises administering to a subject the soluble CTLA4 mutant molecule of the invention to regulate T cell interactions with the CD80 and/or CD86 positive cells. In accordance with the practice of the invention, the soluble CTLA4 mutant molecule can be CTLA4Ig fusion protein. Alternatively, the soluble CTLA4 mutant molecule is a mutant CTLA4 hybrid having a membrane glycoprotein joined to mutant CTLA4.

The present invention also provides method for inhibiting graft versus host disease in a subject. This method comprises administering to the subject the soluble CTLA4 mutant molecule of the invention together with a ligand reactive with IL-4.

The invention encompasses the use of mutant CTLA4 molecules together with other immunosuppressants, e.g., cyclosporin (Mathiesen, Prolonged Survival and Vascularization of Xenografted Human Glioblastoma Cells in the Central Nervous System of Cyclosporin A-Treated Rats Cancer Lett., 44(2), 151-6 (1989), prednisone, azathioprine, and methotrexate (R. Handschumacher "Chapter 53: Drugs Used for Immunosuppression" pages 1264-1276). Other immunosuppressants are possible.

### Expression of CTLA4 mutant molecules in Prokaryotic Cells

Expression of CTLA4 mutant molecules in prokaryotic cells is preferred for some purposes.

Prokaryotes most frequently are represented by various strains of bacteria. The bacteria may be a gram positive or a gram negative. Typically, gram-negative bacteria such as E. coli are preferred. Other microbial strains may also be used.

Sequences encoding CTLA4 mutant molecules can be inserted into a vector designed for expressing foreign sequences in procaryotic cells such as E. coli. These vectors can include commonly used prokaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang et al., Nature 198:1056 (1977)), the tryptophan (trp) promoter system (Goeddel et al., Nucleic Acids Res. 8:4057 (1980)) and the lambda derived P_{L} promoter and N-gene ribosome binding site (Shimatake et al., Nature 292:128 (1981)).

Such vectors will also include origins of replication and selectable markers, such as a beta-lactamase or neomycin phosphotransferase gene conferring resistance to antibiotics so that the vectors can replicate in bacteria and cells carrying the plasmids can be selected for when grown in the presence of ampicillin or kanamycin.

The expression plasmid can be introduced into prokaryotic cells via a variety of standard methods, including but not limited to CaCl₂-shock (see Cohen, Proc. Natl. Acad. Sci. USA (1972) 69:2110, and Sambrook et al. (eds.), Molecular Cloning: A Laboratory Manual, 2nd Edition, Cold Spring Harbor Press, (1989)) and electroporation.

### Expression of CTLA4 mutant molecules in Eukaryotic Cells

In accordance with the practice of the invention, eukaryotic cells are also suitable host cells.

Examples of eukaryotic cells include any animal cell, whether primary or immortalized, yeast (e.g., Saccharomyces cerevisiae, Schizosaccharomyces pombe, and Pichia pastoris), and plant cells. Myeloma, COS and CHO cells are examples of animal cells that may be used as hosts. Exemplary plant cells include tobacco (whole plants or tobacco callus), corn, soybean, and rice cells. Corn, soybean, and rice seeds are also acceptable.

Sequences encoding the CTLA4 mutant molecules can be inserted into a vector designed for expressing foreign sequences in a eukaryotic host. The regulatory elements of the vector can vary according to the particular eukaryotic host.

Commonly used eukaryotic control sequences include promoters and control sequences compatible with mammalian cells such as, for example, CMV promoter (CDM8 vector) and avian sarcoma virus (ASV) (πLN vector). Other commonly used promoters include the early and late promoters from Simian Virus 40 (SV 40) (Fiers, et al., Nature 273:113 (1973)), or other viral promoters such as those derived from polyoma, Adenovirus 2, and bovine papilloma virus. An inducible promoter, such as hMTII (Karin, et al., Nature 299:797-802 (1982)) may also be used.

Vectors for expressing CTLA4 mutant molecules in eukaryotes may also carry sequences called enhancer regions. These are important in optimizing gene expression and are found either upstream or downstream of the promoter region.

Sequences encoding CTLA4 mutant molecules can integrate into the genome of the eukaryotic host cell and replicate as the host genome replicates. Alternatively, the vector carrying CTLA4 mutant molecules can contain origins of replication allowing for extrachromosomal replication.

For expressing the sequences in Saccharomyces cerevisiae, the origin of replication from the endogenous yeast plasmid, the 2µ circle could be used. (Broach, Meth. Enz. 101:307 (1983). Alternatively, sequences from the yeast genome capable of promoting autonomous replication could be used (see, for example, Stinchcomb et al., Nature 282:39 (1979)); Tschemper et al., Gene 10:157 (1980); and Clarke et al., Meth. Enz. 101:300 (1983)).

Transcriptional control sequences for yeast vectors include promoters for the synthesis of glycolytic enzymes (Hess et al., J. Adv. Enzyme Reg. 7:149 (1968); Holland et al., Biochemistry 17:4900 (1978)). Additional promoters known in the art include the CMV promoter provided in the CDM8 vector (Toyama and Okayama, FEBS 268:217-221 (1990); the promoter for 3-phosphoglycerate kinase (Hitzeman et al., J. Biol. Chem. 255:2073 (1980)), and those for other glycolytic enzymes.

Other promoters are inducible because they can be regulated by environmental stimuli or the growth medium of the cells. These inducible promoters include those from the genes for heat shock proteins, alcohol dehydrogenase 2, isocytochrome C, acid phosphatase, enzymes associated with nitrogen catabolism, and enzymes responsible for maltose and galactose utilization.

Regulatory sequences may also be placed at the 3' end of the coding sequences. These sequences may act to stabilize messenger RNA. Such terminators are found in the 3' untranslated region following the coding sequences in several yeast-derived and mammalian genes.

Exemplary vectors for plants and plant cells include but are not limited to Agrobacterium Tᵢ plasmids, cauliflower mosaic virus (CaMV), tomato golden mosaic virus (TGMV).

General aspects of mammalian cell host system transformations have been described by Axel (U.S. Patent No. 4,399,216 issued Aug. 16, 1983). Mammalian cells be transformed by methods including but not limited to, transfection in the presence of calcium phosphate, microinjection, electorporation, or via transduction with viral vectors.

Methods for introducing foreign DNA sequences into plant and yeast genomes include (1) mechanical methods, such as microinjection of DNA into single cells or protoplasts, vortexing cells with glass beads in the presence of DNA, or shooting DNA-coated tungsten or gold spheres into cells or protoplasts; (2) introducing DNA by making protoplasts permeable to macromolecules through polyethylene glycol treatment or subjection to high voltage electrical pulses (electroporation); or (3) the use of liposomes (containing cDNA) which fuse to protoplasts.

### Identification and Recovery of CTLA4 mutant molecules

Expression of CTLA4 mutant molecules is detected by Coomassie stained SDS-PAGE and immunoblotting using antibodies that bind CTLA4. Protein recovery is effected by standard protein purification means, e.g., affinity chromatography or ion-exchange chromatography, to yield substantially pure product (R. Scopes Protein Purification, Principles and Practice, Third Edition Springer-Verlag (1994)).

### CTLA4Ig CODON-BASED MUTAGENESIS

In one embodiment, site-directed mutagenesis and a novel screening procedure were used to identify several mutations in the extracellular domain of CTLA4 that improve binding avidity for CD86, while only marginally altering binding to CD80. In this embodiment, mutations were carried out in residues in the CDR1 loop (serine 25 to arginine 33, the C' strand (alanine 49 and threonine 51), the F strand (lysine 95, glutamic acid 97 and leucine 98), and in CDR3 at positions methionine 99 through tyrosine 104, tyrosine 105 through glycine 109 and in the G strand at positions glutamine 114, tyrosine 116 and isoleucine 118. These sites were chosen based on studies of chimeric CD28/CTLA4 fusion proteins (J. Exp. Med., 1994, 180:2049-2058), and on a model predicting which amino acid residue side chains would be solvent exposed, and a lack of amino acid residue identity or homology at certain positions between CD28 and CTLA4. Also, any residue which is spatially in close proximity (5 to 20 Angstrom Units) to the identified residues are considered part of the present invention.

To synthesize and screen soluble CTLA4 mutant molecules with altered affinities for CD86, a two-step strategy was adopted. The experiments entailed first generating a library of mutations at a specific codon of an extracellular portion of CTLA4 and then screening these by BIAcore analysis to identify mutants with altered reactivity to CD80 or CD86.

### Advantages of the Invention:

Soluble CTLA4 mutant molecules having a higher avidity for CD86 than wild type CTLA4 should be better able to block the priming of antigen specific activated cells than CTLA4Ig.

Further, production costs for CTLA4Ig are very high. High avidity mutant CTLA4Ig molecules that have more potent immunosuppressive properties could be used in the clinic at considerably lower doses than CTLA4Ig to achieve similar levels of immunosuppression. Soluble CTLA4 mutant molecules, e.g., LEA29Y, could be very cost effective.

The following example is presented to illustrate the present invention and to assist one of ordinary skill in making and using the same. This example is not intended in any way to otherwise limit the scope of the invention.

### EXAMPLE 1

Current *in vitro* and *in vivo* studies indicate that CTLA4Ig by itself is unable to completely block the priming of antigen specific activated T cells. *In vitro* studies with CTLA4Ig and either monoclonal antibody specific for CD80 or CD86 measuring inhibition of T cell proliferation indicate that anti-CD80 monoclonal antibody did not augment CTLA4Ig inhibition. However, anti-CD86 monoclonal antibody did, indicating that CTLA4Ig was not as effective at blocking CD86 interactions. These data support earlier findings by Linsley et al. (Immunity, 1994, 1:793-801) showing inhibition of CD80-mediated cellular responses required approximately 100 fold lower CTLA4Ig concentrations than for CD86-mediated responses. Based on these findings, it was surmised that soluble CTLA4 mutant molecules having a higher avidity for CD86 than wild type CTLA4 should be better able to block the priming of antigen specific activated cells than CTLA4Ig.

To this end, site-directed mutagenesis and a novel screening procedure were used to identify several mutations in the extracellular domain of CTLA4 that improve binding avidity for CD86, while only marginally altering binding to CD80. Mutations were carried out in residues in the CDR1 loop (serine 25 to arginine 33, the C' strand (alanine 49 and threonine 51), the F strand (lysine 95, glutamic acid 97 and leucine 98), and in CDR3 at positions methionine 99 through tyrosine 104, tyrosine 105 through glycine 109 and in the G strand at positions glutamine 114, tyrosine 116 and isoleucine 118. These sites were chosen based on studies of chimeric CD28/CTLA4 fusion proteins (J. Exp. Med., 1994, 180:2049-2058), and on a model predicting which amino acid residue side chains would be solvent exposed, and a lack of amino acid residue identity or homology at certain positions between CD28 and CTLA4.

### Methods:

### CTLA4Ia codon based mutagenesis:

Mutagenic oligonucleotide PCR primers were designed for random mutagenesis of a specific codon by allowing any base at positions 1 and 2 of the codon, but only guanine or thymine at position 3 (XXG/T). In this manner, a specific codon encoding an amino acid could be randomly mutated to code for each of the 20 amino acids. PCR products encoding mutations in close proximity to the CDR3-like loop of CTLA4Ig (MYPPPY), were digested with SacI/XbaI and subcloned into similarly cut CTLA4Ig IILN expression vector. For mutagenesis in proximity to the CDR1-like loop of CTLA4Ig, a silent NheI restriction site was first introduced 5' to this loop, by PCR primer-directed mutagenesis. PCR products were digested with NheI/XbaI and subcloned into similarly cut CTLA4Ig expression vector.

### Plasmid miniprep cDNA preparation:

Ninety six transformed bacterial colonies, each representing a single mutant at a specific site were grown and cDNA robotically prepared using a Biorobot 9600 (Qiagen).

### COS cell transfection:

COS cells grown in 24 well tissue culture plates were transiently transfected with mutant CTLA4Ig and culture media collected 3 days later.

### BIAcore analysis:

Conditioned COS cell culture media was allowed to flow over BIAcore biosensor chips derivitized with CD86Ig or CD80Ig, and mutant molecules were identified with off rates slower than that observed for wild type CTLA4Ig. cDNA corresponding to selected media samples were sequenced and enough DNA prepared to perform larger scale COS cell transient transfection, from which mutant CTLA4Ig protein was prepared following protein A purification of culture media.

BIAcore analysis conditions and equilibrium binding data analysis were performed as described in J. Greene et al. (1996) JBC 271(42):26762.

BIAcore Data Analysis: Senosorgram baselines were normalized to zero response units (RU) prior to analysis. Samples were run over mock derivatized flow cells to determine background RU values due to bulk refractive index differences between solutions. Equilibrium dissociation constants (K_{d}) were calculated from plots of R_{eq} versus C, where R_{eq} is the steady-state response minus the response on a mock-derivatized chip, and C is the molar concentration of analyte. Binding curves were analyzed using commercial nonlinear curve-fitting software (Prism, GraphPAD Software).

Experimental data were first fit to a model for a single ligand binding to a single receptor (1-site model, i.e., a simple langmuir system, A+BÑAB), and equilibrium association constants (K_{d}=[A] .[B]\[AB]) were calculated from the equation R=Rₘₐₓ.C/(K_{d}+C). Subsequently, data were fit to the simplest two-site model of ligand binding (i.e., to a receptor having two non-interacting independent binding sites as described by the equation R=Rₘₐₓ₁.C\(K_{d1}+C)+R ₘₐₓ₂.C\(K_{d2}+C).

The goodness-of-fits of these two models were analyzed visually by comparison with experimental data and statistically by an F test of the sums-of-squares. The simpler one-site model was chosen as the best fit unless the two-site model fit significantly better (p<0.1).

Association and disassociation analyses were performed using BIA evaluation 2.1 Software (Pharmacia). Association rate constants kₒₙ were calculated in two ways, assuming both homogenous single-site interactions and parallel two-site interactions. For single-site interactions, kₒₙ values were calculated according to the equation Rₜ=R_{eq}(1-exp^{-ks(t-t}₀⁾ , where Rₜ is a response at a given time, t; R_{eq} is the steady-state response; to is the time at the start of the injection; and kₛ=dR/dt=kₒₙ.Ck_{off}, where C is a concentration of analyte, calculated in terms of monomeric binding sites. For two-site interactions kₒₙ values were calculated according to the equation Rₜ=R_{eq1} (1-exp^{-ks1(t-t}₀)+R_{eq2}(1-exp^{ks2(t-t}₀⁾. For each model, the values of kₒₙ were determined from the calculated slope (to about 70% maximal association) of plots of kₛ versus C.

Dissociation data were analyzed according to one site (AB=A+B) or two sites (AiBj=Ai+Bj) models, and rate constants (k_{off}) were calculated from best fit curves. The binding site model was used except when the residuals were greater than machine background (2-10RU, according to machine), in which case the two-binding site model was employed. Half-times of receptor occupancy were calculated using the relationship t_{1/2}=0.693/k_{off}.

### Flow Cytometry:

Murine MAb L307.4 (anti-CD80) was purchased from Becton Dickinson (San Jose, California) and IT2.2 (anti-B7-0[CD86]), from Pharmingen (San Diego, California). For immunostaining, CD80 and/or CD86 +CHO cells were removed from their culture vessels by incubation in phosphate-buffered saline containing 10mM EDTA. CHO cells (1-10 x 10⁵) were first incubated with MAbs or immunoglobulin fusion proteins in DMEM containing 10% fetal bovine serum (FBS), then washed and incubated with fluorescein isothiocyanate-conjugated goat anti-mouse or anti-human immunoglobulin second step reagents (Tago, Burlingame, California). Cells were given a final wash and analyzed on a FACScan (Becton Dickinson).

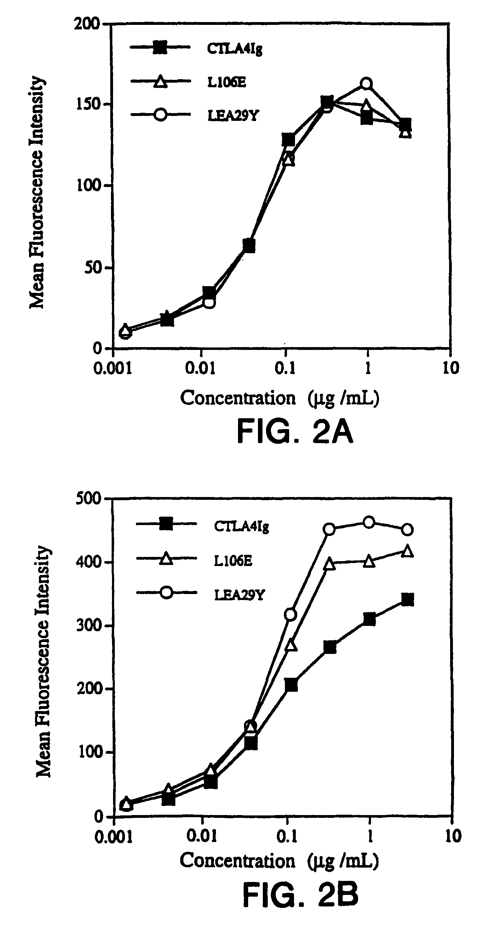

FACS analysis (Fig. 2) of CTLA4Ig and mutant molecules binding to stably transfected CD80+ and CD86+CHO cells was performed as described herein.

CD80+ and CD86+ CHO cells were incubated with increasing concentrations of CD28Ig, washed and bound immunoglobulin fusion protein was detected using fluorescein isothiocyanate-conjugated goat anti-human immunoglobulin. Binding of CTLA4Ig was also measured using the same procedure.

In Figure 2 LEA29Y (circles) and L106E (triangle) CHO cells (1.5x10⁵) were incubated with the indicated concentrations of CTLA4Ig (closed square) for 2 hr. at 23°C, washed, and incubated with fluorescein isothiocyanate-conjugated goat anti-human immunoglobulin antibody. Binding on a total of 5,000 viable cells was analyzed (single determination) on a FACScan, and mean fluorescence intensity (MFI) was determined from data histograms using PC-LYSYS. Data have been corrected for background fluorescence measured on cells incubated with second step reagent only (MFI = 7). Control L6 MAb (80 µg/ml) gave MFI < 30. This is representative of four independent experiments.

### Functional assays:

Human CD4+ T cells were isolated as described herein.

CD4⁺T cells were isolated by immunomagnetic negative selection (Linsley et al., (1992 "Coexpression and functional cooperativity of CTLA4 and CD28 on activated T lymphocytes" J. Exp. Med. 176:1595-1604).

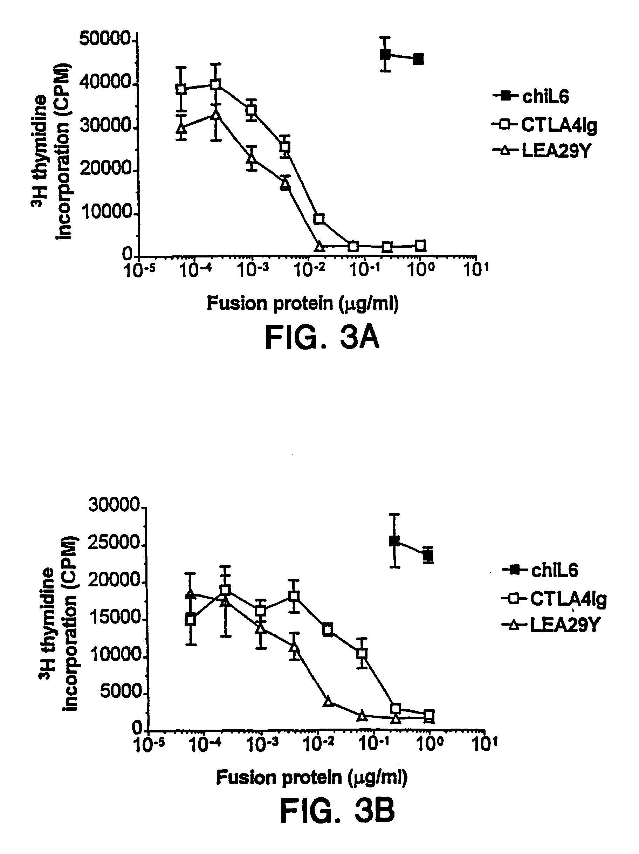

Inhibition of PMA plus CD80-CHO or CD86-CHO T cell stimulation (Fig. 3) was performed. For stimulation assays, PHA blasts (Linsley et al., (1991) "Binding of the B cell activation antigen B7 to CD28 costimuates T cell proliferation and IL-2 mRNA accumulation" J. Exp. Med. 173:561-570) were cultured at 4 x 10⁴/well with or without irradiated CHO cell stimulators. CD4⁺T cells (8-10 x 10⁴/well) were cultured in the presence of 1 nM PMA with or without irradiated CHO cell stimulators. Proliferative responses were measured by the addition of 1 µCi/well of [ ³H]thymidine during the final 7 hr. of a 72 hr. culture. IL-2 production in conditioned medium (collected after 24 hr. stimulation) was measured by enzyme immunoassay (Biosource, Camarillo, California).

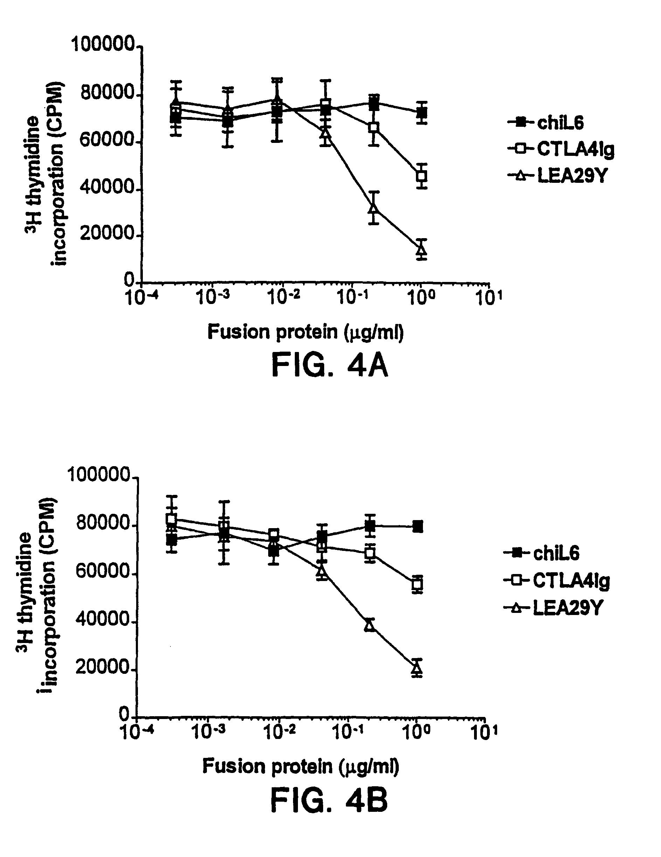

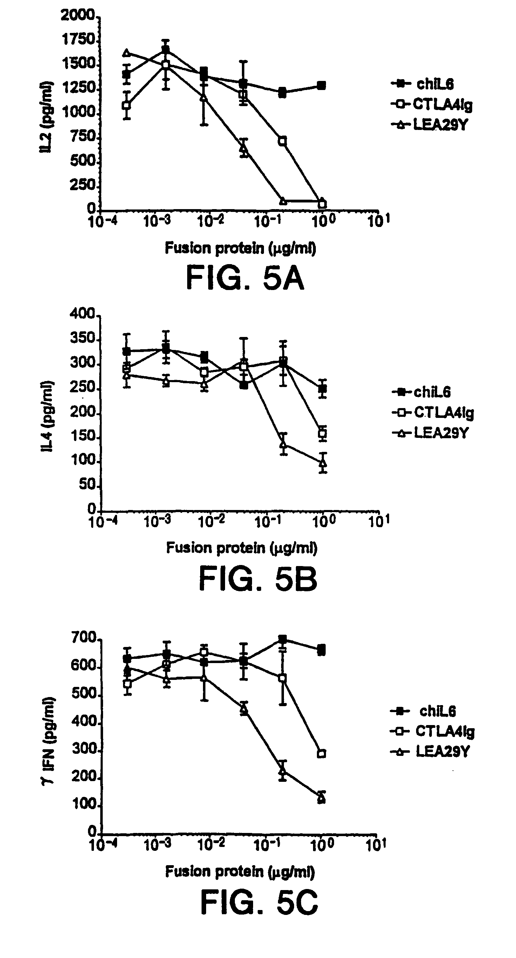

Figures 4 and 5 show inhibition of allostimulated human T cells prepared above, and allostimulated with a human B LCL line called PM. T cells at 3.0x10⁴/well and PM at 8.0x10³ /well. Primary allostimulation for 6 days then cells pulsed with ³H-thymidine for 7 hours before incorporation of radiolabel was determined. Secondary allostimulation performed as follows. Seven day primary allostimulated T cells were harvested over LSM (Ficol) and rested for 24 hours. T cells then restimulated (secondary) by adding PM in same ratio as above. Stimulate 3 days, pulse with radiolabel and harvest as above. To measure cytokine production (Fig. 5), duplicate secondary allostimulation plates were set up. After 3 days, culture media was assayed using Biosource kits using conditions recommended by manufacturer.

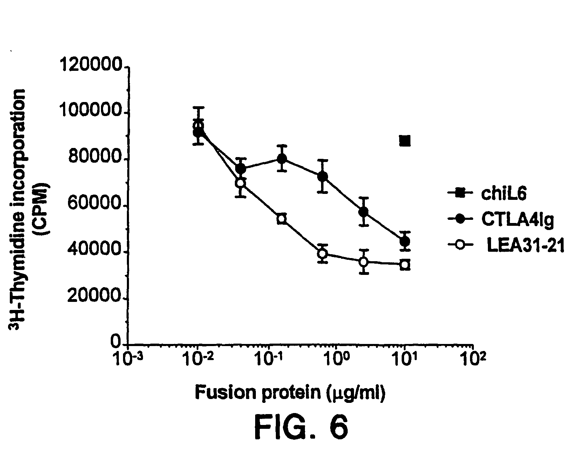

Monkey MLR (Fig. 6). PBMC'S from 2 monkeys purified over LSM and mixed (3.5x10⁴ cells/well from each monkey) with 2ug/ml PHA. Stimulated 3 days then pulsed with radiolabel 16h before harvesting.

**Table I**

| Equilibrium binding constants. | | |
|---|---|---|
| | **CD80Ig (Kd)** | **CD86Ig (Kd)** |
| **CTLA4Ig** | 0.925"0.025 | 5.2"1.38 |
| **L106E** | 0.84"0.04 | 3.4"0.35 |
| **LEA29Y** | 1.26"0.34 | 2.6"0.71 |

BIAcore™ Analysis: All experiments were run on BIAcore™ or BIAcore™ 2000 biosensors (Pharmacia Biotech AB, Uppsala) at 25°C. Ligands were immobilized on research grade NCM5 sensor chips (Pharmacia) using standard N-ethyl-N'-(dimethylaminopropyl) carbodiimidN-hydroxysuccinimide coupling (Johnsson, B., et al. (1991) Anal. Biochem. 198: 268-277; Khilko, S.N., et al.(1993) J. Biol. Chem 268: 5425-15434).

SEQUENCE LISTING

(1) GENERAL INFORMATION:

(i) APPLICANTS: Peach, Robert J.
Namura, Joseph R.
Linsley, Peter S.
Bajorath, Jurgen

(ii) TITLE OF INVENTION: SOLUBLE CTLA4 MUTANT MOLECULES
AND USES THEREOF

(iii) NUMBER OF SEQUENCES: 1

(iv) CORRESPONDENCE ADDRESS:
(A) ADDRESSEE: Bristol-Myers Squibb Company
(B) STREET: P.O. Box 4000
(C) CITY: Princeton,
(D) STATE: New Jersey
(E) COUNTRY: United States
(F) ZIP: 08543-4000

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: Sorrentino, Joseph M.
(B) REGISTRATION NUMBER: 32,598
(C) REFERENCE/DOCKET NUMBER: ON0152a

(ix) TELECOMMUNICATION INFORMATION:
(A) TELEPHONE: (609) 252-3953
(B) TELEFAX: (609) 252-4526

(1) INFORMATION FOR SEQ ID NO:1:

(i) SEQUENCE CHARACTERISTICS:
(A) LENGTH: 561 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens

(ix) FEATURE:
(A) NAME/KEY: CDS
(B) LOCATION: 1..561

(xi) SEQUENCE DESCRIPTION: SEQ ID NO:1:

GCA ATG CAC GTG GCC CAG CCT GCT GTG GTA CTG GCC AGC AGC CGA
GGC 48
Ala Met His Val Ala Gln Pro Ala Val Val Leu Ala Ser Ser Arg
Gly
1 5 10 15

ATC GCC AGC TTT GTG TGT GAG TAT GCA TCT CCA GGC Xaa GCC ACT
GAG 96
Ile Ala Ser Phe Val Cys Glu Tyr Ala Ser Pro Gly Xaa Ala Thr
Glu
20 25 30

GTC CGG GTG ACA GTG CTT CGG CAG GCT GAC AGC CAG GTG ACT GAA
GTC 144
Val Arg Val Thr Val Leu Arg Gln Ala Asp Ser Gln Val Thr Glu
Val
35 40 45

TGT GCG GCA ACC TAC ATG ATG GGG AAT GAG TTG ACC TTC CTA GAT
GAT 192
Cys Ala Ala Thr Tyr Met Met Gly Asn Glu Leu Thr Phe Leu Asp
Asp
50 55 60

TCC ATC TGC ACG GGC ACC TCC AGT GGA AAT CAA GTG AAC CTC ACT
ATC 240
Ser Ile Cys Thr Gly Thr Ser Ser Gly Asn Gln Val Asn Leu Thr
Ile
65 70 75
80

CAA GGA CTG AGG GCC ATG GAC ACG GGA CTC TAC ATC TGC AAG GTG
GAG 288
Gln Gly Leu Arg Ala Met Asp Thr Gly Leu Tyr Ile Cys Lys Val
Glu
85 90 95

CTC ATG TAC CCA CCG CCA TAC TAC CTG Yaa ATA GGC AAC GGA ACC
CAG 336
Leu Met Tyr Pro Pro Pro Tyr Tyr Leu Yaa Ile Gly Asn Gly Thr
Gln
100 105 110

ATT TAT GTA ATT GAT CCA GAA CCG TGC CCA GAT TCT GAC TTC CTC
CTC 384
Ile Tyr Val Ile Asp Pro Glu Pro Cys Pro Asp Ser Asp Phe Leu
Leu
115 120 125

TGG ATC CTT GCA GCA GTT AGT TCG GGG TTG TTT TTT TAT AGC TTT
CTC 432
Trp Ile Leu Ala Ala Val Ser Ser Gly Leu Phe Phe Tyr Ser Phe
Leu
130 135 140

CTC ACA GCT GTT TCT TTG AGC AAA ATG CTA AAG AAA AGA AGC CCT
CTT 480
Leu Thr Ala Val Ser Leu Ser Lys Met Leu Lys Lys Arg Ser Pro
Leu
145 150 155
160

ACA ACA GGG GTC TAT GTG AAA ATG CCC CCA ACA GAG CCA GAA TGT
GAA 528
Thr Thr Gly Val Tyr Val Lys Met Pro Pro Thr Glu Pro Glu Cys
Glu
165 170 175

AAG CAA TTT CAG CCT TAT TTT ATT CCC ATC AAT
561
Lys Gln Phe Gln Pro Tyr Phe Ile Pro Ile Asn
180 185

## Claims

1. A soluble CTLA4 mutant molecule having a higher avidity for CD86 than wildtype CTLA4, comprising an extracellular domain of CTLA4 having one or more mutations selected from the group consisting of mutations in the CDR1 loop (serine 25 to arginine 33, the C' strand (alanine 49 to threonine 51), the F strand (lysine 95, glutamic acid 97 and leucine 98), and in CDR3 at positions methionine 99 through tyrosine 104, tyrosine 105 through glycine 109 and in the G strand at positions gutamine 114, tyrosine 116 and isoleucine 118.

2. The CTLA4 mutant molecule according to claim 1, wherein wildtype CTLA4 is naturally occurring CTLA4.

3. The CTLA4 mutant molecule according to claim 1, which binds with higher avidity to CD86 than CTLA4Ig.

4. The CTLA4 mutant molecule according to claim 1, further comprising a biologically or chemically active non-CTLA4 molecule.

5. The CTLA4 mutant molecule according to claim 1, which is a mutant CTLA41g fusion protein.

6. A nucleic acid molecule encoding the amino acid sequence corresponding to the CTLA4 mutant molecule of any one of claims 1 to 5.

7. The nucleic acid molecule according to claim 6, which is cDNA.

8. A plasmid comprising the nucleic acid molecule of claim 7.

9. A host vector system comprising the plasmid of claim 8 in a suitable host cell.

10. The host vector system according to claim 9, wherein the suitable host cell is a bacterial cell or a eukaryotic cell.

11. A method for producing a protein comprising growing the host vector system of claim 11 so as to produce the protein in a host, and recovering the protein so produced.

12. A protein obtainable by the method of claim 11.

13. A pharmaceutical composition comprising the soluble CTLA4 mutant molecule of any one of claims 1 to 5 or the protein of claim 12.

14. An in vitro method for regulating functional CTLA4 and CD28 positve T cell interactions with CD80 and/or CD86 positive cells, comprising contacting the CD80 and/or CD86 positive cells with the soluble CTLA4 mutant molecule of any one of claims 1 to 5 or the protein of claim 12 so as to form CTLA4/CD80 and/or CTLA4/CD86 complexes, the complexes interfering with reaction of endogenous CTLA4 antigen with CD80 and/or CD86.

15. The use of the soluble CTLA4 mutant molecule of any one of claims 1 to 5 or the protein of claim 12 for preparing a pharmaceutical composition for immune suppression.

16. The use of the soluble CTLA4 mutant molecule of any one of claims 1 to 5 or the protein of claim 12 for preparing a pharmaceutical composition for treating an immune system disease.

17. The use according to claim 16, wherein the immune system disease is mediated by CD28 and/or CTLA4 positive cell interactions with dentritic cells with CD86/CD80 positive cells.

18. The use according to claim 17, wherein said T cell interactions are inhibited.

19. The use of the soluble CTLA4 mutant molecule of any one of claims 1 to 5 or the protein of claim 12 and a ligand reactive with IL-4 for preparing a pharmaceutical composition for inhibiting graft versus host disease (GVHD).

20. Immunosuppressive combination comprising the soluble CTLA4 mutant molecule of any one of claims 1 to 5 or the protein of claim 12 and at least one further immunosuppressant selected from the group consisting of cyclosporin, prednisone, azathioprine and methotrexate.
